Europäisches Patentamt

European Patent Office

Office européen des brevets

(11) Publication number: **0 161 063 B1**

# EUROPEAN PATENT SPECIFICATION

(45) Date of publication of patent specification: **24.07.91**   (51) Int. Cl.⁵: **A61K 37/38**

(21) Application number: **85302353.9**

(22) Date of filing: **03.04.85**

The file contains technical information submitted after the application was filed and not included in this specification

(54) Method of inducing ovulation.

(30) Priority: **03.04.84 US 596384**

(43) Date of publication of application:
**13.11.85 Bulletin 85/46**

(45) Publication of the grant of the patent:
**24.07.91 Bulletin 91/30**

(84) Designated Contracting States:
**AT BE CH DE FR GB IT LI LU NL SE**

(56) References cited:
**US-A- 4 317 815**
**US-A- 4 431 635**

**CHEMICAL ABSTRACTS, vol. 100, no. 25, 18th June 1984, page 98, no. 203981b, Columbus, Ohio, US; R.S.SCHENKEN et al.: "Ovulation induction using "pure" follicle-stimulating hormone in monkeys"**

(73) Proprietor: **SERONO LABORATORIES, INC.**
**280 Pond Street**
**Randolph Massachusetts(US)**

(72) Inventor: **Hodgen, Gary D.**
**619 Mobray Arch**
**Norfolk, Virginia(US)**

(74) Representative: **Lawrence, Peter Robin Broughton**
**GILL JENNINGS & EVERY 53-64 Chancery Lane**
**London WC2A 1HN(GB)**

## Description

It is accepted dogma that typically ovulation of a single, fertilisable ovum each menstrual cycle completes a course of oogenesis that began during fetal development. It is not known, however, why from among the thousands of follicles present from birth, only a relative few are recruited each cycle to grow while at the same time others remain at rest. It is also not known why from the host of follicles maturing in each ovary, typically only a single follicle escapes atresia and is selected to ovulate each cycle. Since the vast majority of follicles fall victim to atresia (greater than 99%), understanding the selection of the follicle destined to ovulate is fraught with the inherent difficulty of studying the rare exception rather than the predominant rule. One must be extremely careful to distinguish follicle growth that culminates in ovulation (gametogenic follicle growth) from that ending in atresia.

The latter stages of oogenesis in adults (i.e., folliculogenesis) are known to depend, to a large degree, on a complex interplay of hormones from the hypothalamus, pituitary and ovary. However, even though much more is understood about these endocrine relationships today, what determines the fate of an individual follicle remains largely unknown. Since in higher primates both ovaries are functional, the maturation of a single follicle with the potential to ovulate brings with it the obvious concomitant of one active and one quiescent ovary each cycle. It is not known how just a single follicle matures to ovulation on only one ovary each cycle, even though both ovaries are perfused by a common systemic circulation.

Three glycoprotein hormones, (luteinizing hormone (or LH), follicle stimulating hormone (or FSH) and human chorionic gonadotropin (or hCG)) act on the ovary to stimulate steroid synthesis and secretion. LH and FSH are secreted by the pituitary and together play a central role in regulating the menstrual cycle and ovulation. hCG is secreted by the developing placenta from the early stages of pregnancy and its role is to maintain steroid secretion by the corpus luteum, which is necessary to prevent ovulation during pregnancy and to preserve the conceptus during early pregnancy.

In the normal cycle, there is a mid-cycle surge in LH concentration which is followed by ovulation. An elevated estrogen level, which is brought about by the endogenous secretion of LH and FSH, is required for the LH surge to occur. The estrogen mediates a positive feedback mechanism which results in the increased LH secretion.

It is now known how to employ exogeneous hormonal stimulation by administering mixed human menopausal gonadotropins, i.e., a combination of FSH and LH, as a prelude to ovulation or follicle aspiration for oocyte collection in in vitro fertilisation techniques. Women and monkeys treated with such human menopausal gonadotropins often fail to demonstrate a timely LH surge despite serum estradiol levels sufficient to elicit positive feedback of LH secretion. It has been concluded that the human menopausal gonadotropins stimulate the production of an ovarian factor or factors which block the pituitary LH response to the gonadotropin releasing hormone (GnRH). This blockage of GnRH action on the pituitary may be the mechanism by which the human menopausal gonadotropin stimulation prevents the estrogen mediated positive feedback of LH secretion. Non-human primates have been employed in research because of their extensive mimicry of many anatomic, functional and temporal characteristics of the hypothalamic-pituitary-ovarian-uterine axis in women. The individual variation in serum estrogen levels of endocrine normal individuals in response to human menopausal gonadotropin stimulation, as seen in these primates, is well recognised clinically. This has resulted in the adoption of an individualised regimen for ovulation induction by human menopausal gonadotropin/hCG. Although LH surges do occur spontaneously, their appearance is sufficiently infrequent that hCG is routinely administered to induce ovulation.

Administration of human menopausal gonadotropins to ovulatory monkeys produces familiar bilateral ovarian hyperstimulation with attendant super physiological elevations of circulating estradiol. Despite these elevated estrogen levels, the monkeys failed to manifest timely gonadotropin responses to estrogen positive feedback, i.e., stereotypically these normal, intact, cycling primates do not have the expected mid-cycle-like LH surges despite escalating levels of serum estradiol that usually exceed 400 pg/ml during 12 days of human menopausal gonadotropin therapy. An absence of spontaneous LH surges has also been observed when human menopausal gonadotropin induced ovarian hyperstimulation occurs in post-partum monkeys. These observations fit in with the frequent clinical finding that when endocrinologically normal patients are given human menopausal gonadotropins to increase the number of follicles/ova available for in vitro fertilisation and embryo transfer therapy, hCG is usually required for the final maturation of these follicles.

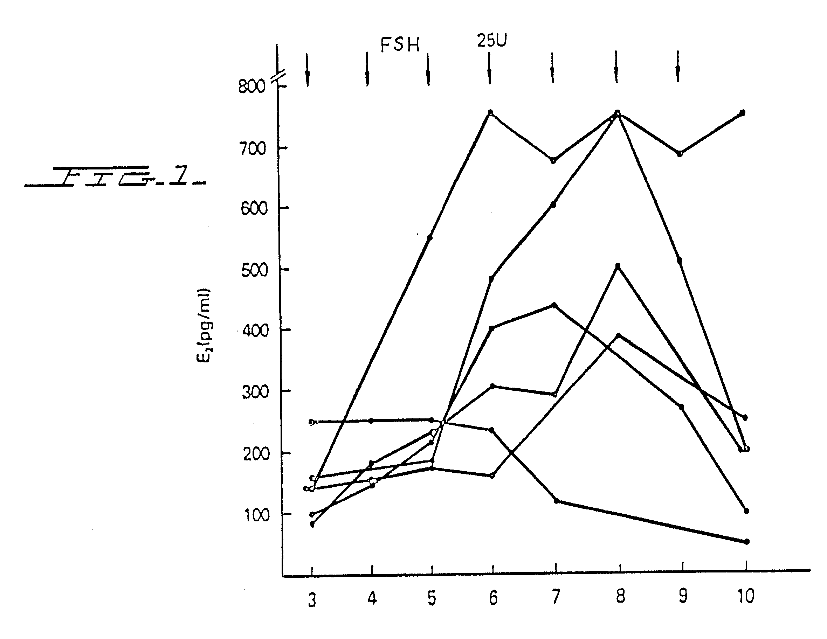

It is well established that the appropriate application of mixed exogeneous gonadotropins has proved efficacious for ovulation induction or for multiple egg retrieval during in vitro fertilisation therapy in women. However, ovarian stimulation through exogeneous gonadotropins for in vivo and in vitro fertilisation therapy is notoriously difficult to manage and the lack of uniform success with conventional human menopausal gonadotropin medications, those containing FSH and LH in nearly equal amounts, is widely appreciated. Individual responses to human menopausal gonadotropins vary markedly, thereby complicating patient management even when the most flexible (individualised) protocols are used. Figure 1 charts serum estrogen levels of normal cycling monkeys who had 25 IU FSH administered intramuscularly daily on days 3 to 9 after the onset of menses and illustrates the marked individual variability in response to the agent.

It is the object of this invention to provide an improved method of inducing ovulation by the administration of exogeneous human menopausal gonadotropins which increases the likelihood of more uniform follicular maturation or ovulation and is therefore of particular use in connection with in vivo and in vitro fertilisation.

According to the present invention FSH which is substantially free of LH is used in the manufacture of a composition for use in a method of inducing ovulation in conjunction with a gonadotropin releasing hormone antagonist which is administered in an amount sufficient to suppress endogeneous pituitary menopausal gonadotropin secretion.

In addition, according to the present invention a gonadotropin releasing hormone antagonist is used in the manufacture of a composition for use in a method of inducing ovulation by the administration of FSH in the substantial absence of LH.

The compositions may contain both FSH and the gonadotropin releasing hormone antagonist, but preferably the two components are in separate compositions.

Further according to the present invention human chorionic gonadotropin is used in the manufacture of a composition for use in a method of inducing ovulation in conjunction with FSH substantially free of LH and a gonadotropin releasing hormone antagonist.

The compositions are used in the induction of follicular maturation or ovulation by the administration of FSH in the absence of exogeneous LH and are therefore employed in treating infertility syndrome. The individual variations in estrogen response to FSH are mitigated by the cojoint administration of FSH and the GnRH antagonist.

It has been found that the administration of exogeneous FSH in the absence of exogeneous LH is capable of inducing the development of multiple ovarian follicles which are responsive to hCG.

In this novel method of inducing ovulation FSH is used in the same quantities as in the conventional exogeneous FSH/LH combinations. Generally, the amount of FSH administered daily to a woman being treated will be in the range of about 75-225 IU and preferably in the range of about 1.5-4.0 IU/kg/day. Intramuscular injection can be employed.

In order to decrease the marked individual variability in response to human menopausal gonadotropin therapy, a gonadotropin releasing hormone antagonist is administered in order to eliminate endogeneous pituitary FSH and LH secretion. Typical GnRH antagonists are described in Rees et al, J.Med. Chem., 17, 1016 (1974), Coy et al, Peptides 1976 (Loffed Ed., Editions de L'Universite de Bruxelle 1977) p.463, Beattie et al, J.Med. Chem., 18, 1247 (1975), Channabasavaiah et al, Biochem. Biophys. Res. Commun., 86, 1266 (1979) and U.S. Patents 4,317,815 and 4,431,635, and include (Ac-pClPhe$^1$, pClPhe$^2$, DTrp$^3$, DArg$^6$, DAla$^{10}$)GnRH HCl, [D-Phe$^2$]-LHRH, [D-Phe$^2$, D-phe$^6$]-LHRH, [D-Phe$^2$, Phe$^3$, D-Phe$^6$]-LHRH, [D-Phe$^2$, D-Trp$^3$, D-Phe$^6$]-LHRH, [D-p-F-Phe-D-Ala$^6$]-LHRH, and [Ac-D-Phe$^1$, D-Phe$^2$, D-Trp$^{3,6}$]-LHRH.

The GnRH antagonist is administered in an amount which is sufficient to suppress endogeneous gonadotropin secretion. In general, the average daily dosage will be in the range of about 1.0-3.0 mg per kg and preferably in the range of about 1.5-2.5 mg/kg. Intramuscular injection can be employed. The amount of the antagonist and FSH will best be determined by the attending clinician for the individual being treated. This is particularly true for the GnRH antagonist since the various analogs have different potencies.

Figure 1 graphs estradiol concentration as a function of the administration of human menopausal gonadotropin for a set of normal cycling monkeys.

Figure 2 charts estradiol, LH and FSH concentration in intact cycling monkeys which have been treated with a GnRH antagonist and by human menopausal gonadotropin therapy as described in the following example.

Figure 3 graphs estradiol, LH and FSH concentration in intact cycling monkeys to whom a GnRH antagonist and by FSH therapy has been administered as described in the following examples.

EXAMPLE

Eleven adult female cynomolgus monkeys (Macaca fascicularis) were selected for study based on records indicating regular menstrual cycles. The average body weight for these primates was 4.79±0.86 kg.

Counting the first day of spontaneous menses as cycle day 1, the monkeys were treated with 25 IU (im) of FSH twice daily according to three regimens. Group 1 received injections on cycle days 1-11; Group 2 on cycle days 1-4 and Group 3 on days 8-11. For all monkeys, laparoscopies were performed under ketamine anesthesia, beginning on the first day of FSH treatment and serially every 3 to 5 days thereafter to assess the status of ovarian follicular development. In order to test whether these FSH driven follicles could be ovulated, monkeys in Group 1 only received 1,000 IU (im) of hCG on day 12 and retrograde lavage of the fallopian tubes for egg collection 72 hours after hCG treatment was employed to determine whether ovulation had actually occurred. Daily femoral blood samples were collected beginning on day 1 of the cycle and continued for 40 days or until menstruation. Sera were frozen until radioimmunoassay of LH, FSH, $17\beta$ - estradiol and progesterone.

The injections of FSH on cycle days 1-11 induced dramatic and sustained elevations in serum FSH (about 15 $\mu$g/ml) and estradiol (about 500 pg/ml). Concurrently, ovarian hyperstimulation was manifested by obvious multiple follicular growth (10-15 prominent follicles by cycle day 8-11). Prior to hCG treatment, serum LH and progesterone remained at basal levels. Within 48 hours after HCG treatment, 1-3 ovulatory stigma were observed on each ovary. Mean serum progesterone and estradiol levels exceed 15 ng/ml and 400 pg/ml, respectively, in mid-luteal phase, indicative of the collective secretory actions of multiple corpora lutea. That ovulation had actually occurred was indicated by the recovery of one or more eggs from the fallopian tubes of each female.

The monkeys treated with FSH during only the early follicular phase of the menstrual cycle demonstrated a prompt increase in serum FSH concentrations (mostly exogenous) with mean levels near 15 $\mu$g/ml on day 4. On discontinuation of FSH injections, circulating FSH levels declined precipitously, below the limits of detection in radio-immunoassay (cycle days 8-11). Serum estradiol levels increased in parallel with the initial increase in circulating FSH, with mean peak values exceeding 300 pg/ml on cycle days 4-6 but even so, no LH surges were observed. Serum progesterone levels remained basal until the onset of the luteal phase in the subsequent spontaneous ovulatory cycle (day 24±2.4). Laparoscopy prior to FSH treatment reveal no advanced follicular development, while on cycle day 5, both ovaries were enlarged with multiple vesicular follicles. Following withdrawal of FSH treatment, the ovaries gradually returned to normal size over the subsequent week. No ovulatory stigma appeared.

Brief administration of FSH on cycle days 8-11 increased serum FSH levels similar to those found in Groups 1 and 2. Mean serum estradiol concentrations increased abruptly, but a spontaneous LH surge was present in only one of four monkeys. The follicular phase serum hormonal profiles for the solitary female were indistinguishable from those of an untreated ovulatory cycle.

The foregoing results show that FSH can be administered alone to enhance the natural ovarian cycle.

The GnRH antagonist (Ac-pClPhe[1],pClPhe[2], DTrp[3], DArg[6] , DAla[10])GnRH HCl, was administered to 20 normal cynomolgus monkeys (1.0 mg/kg/day) in order to eliminate endogenous pituitary FSH and LH secretion. Superimposed on this treatment, 15 of the monkeys were administered a human menopausal gonadotropin preparation containing approximately equal amounts of FSH and LH and five monkeys were administered FSH only to stimulate ovarian follicular maturation. Control groups received either the combined exogeneous gonadotropins or FSH only (15 monkeys each group). All of the groups received a fixed regimen of gonadotropin therapy for seven consecutive days (25 IU/day/im, cycle days 3-9) followed by hCG (1,000 IU/im) on cycle day 10.

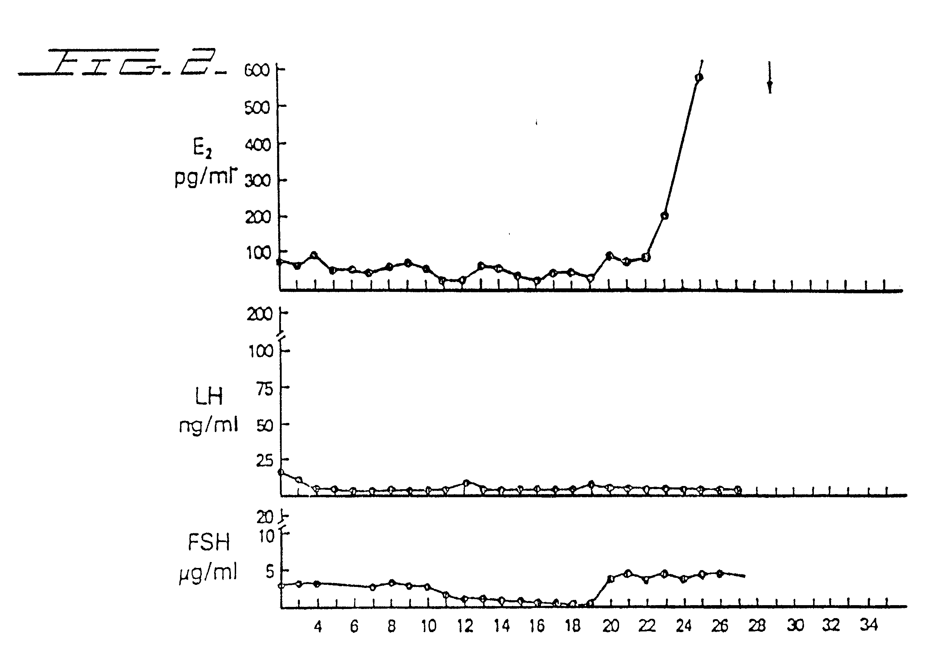

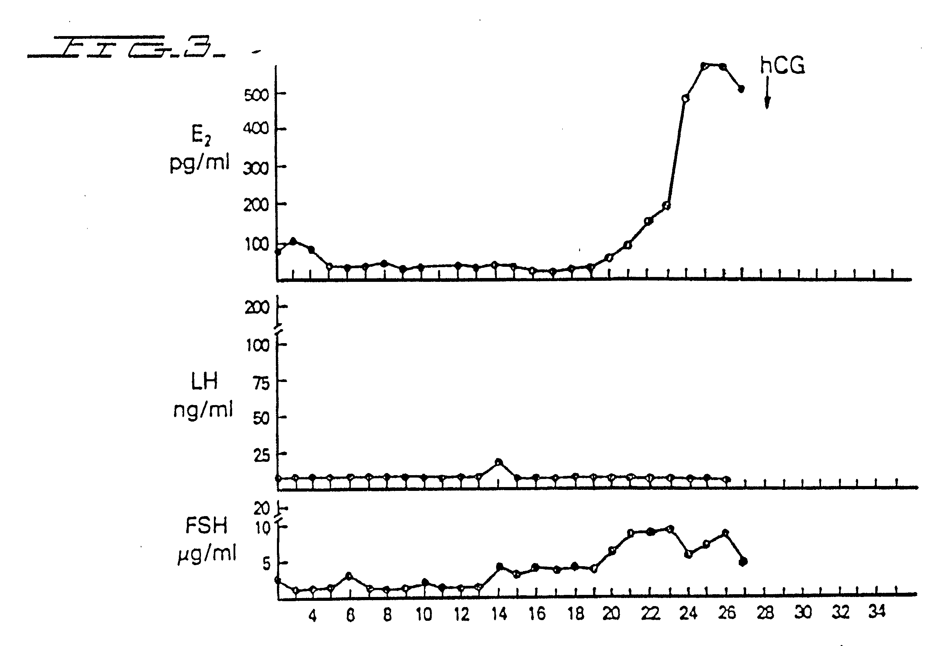

It was observed that the variability in response to gonadotropin treatment was less in the group treated with the GnRH antagonist. The patterns of estradiol rise in serum and the appearance of the ovaries at laparotomy were more uniform when the endogeneous gonadotropin secretion was suppressed. Figures 2 and 3 show assay results. While both treatments suppressed the estrogen variability, only the regimen employing the GnRH antagonist and FSH in the absence of LH promoted maturation of multiple ovarian follicles and these follicles were ovulated by hCG with subsequent recovery of two-cell embryos. This is the first time that ovarian follicular maturation has been demonstrated to be promoted by FSH in the absence of detectable levels of LH in serum. Ovulation was also achieved in 9 of the 15 control monkeys treated with only FSH.

## Claims

1. Use of a gonadotropin releasing hormone antagonist to prepare a pharmaceutical composition for use with a pharmaceutical composition comprising a gonadotropin selected from a) human menopausal gonadotropin and b) FSH substantially free of LH in the treatment of female infertility to suppress estrogen variability, in which treatment the antagonist composition is administered in an effective amount cojointly with the gonadotropin composition.

2. Use of a gonadotropin selected from a) human menopausal gonadotropin and b) FSH substantially free of LH to prepare a pharmaceutical composition for use with a pharmaceutical composition comprising a gonadotropin releasing hormone antagonist in the treatment of female infertility to suppress estrogen variability, in which treatment the antagonist composition is administered in an effective amount cojointly with the gonadotropin composition.

3. Use of a gonadotropin releasing hormone antagonist and a gonadotropin selected from a) human menopausal gonadotropin and b) FSH substantially free of LH to prepare a pharmaceutical preparation for use in the treatment of female infertility to suppress estrogen variability by co-joint administration of an effective amount of a composition comprising gonadotropin releasing hormone antagonist and a composition comprising gonadotropin.

4. Use according to any preceding claim wherein the gonadotropin composition comprises human menopausal gonadotropin that contains approximately equal amounts of FSH and LH.

5. Use according to any of the preceding claims wherein the antagonist and the gonadotropin for the cojoint use are in separate compositions.

6. Use according to any of the preceding claims wherein the composition containing antagonist is in a form suitable for administering a daily dose of 1.0 to 3.0 mg antagonist per kg body weight.

7. Use according to any of the preceding claims wherein the antagonist is (Ac-pclPhe$^1$, pClPhe$^2$, D-Trp$^3$, D-Arg$^6$, D-Ala$^{10}$)GnRH, HCl.

8. Use according to any preceding claim wherein the gonadotropin-containing composition is in a form suitable for administering a daily dose containing 75 to 225 I.U. FSH.

9. Use according to claim 8 in which the composition is in a form suitable for administering a daily dose containing 1.5 to 4.0 IU FSH per kg body weight.

10. Use according to any of the preceding claims in which the, either or each composition is provided in a form suitable for parenteral administration.

11. Use according to any preceding claim in which the treatment includes the use of a pharmaceutical composition containing human chorionic gonadotropin for inducing ovulation.

12. Use according to any of the preceding claims wherein the treatment is for in vitro fertilisation.

13. A pharmaceutical preparation containing gonadotropin selected from a) human menopausal gonadotropin and b) FSH substantially free cf LH and a gonadotropin releasing hormone antagonist as a combined preparation for co-joint use to suppress estrogen variability in a method of treatment of female infertility, in which treatment the antagonist composition is administrered in an effective amount cojointly with the gonadotropin composition.

14. A preparation according to claim 13 in which the gonadotropin composition comprises human menopausal gonadotropin that contains approximately equal amounts of FSH and LH.

15. A preparation according to any of the claims 13 to 14 in which the antagonist and the gonadotropin are in the same or separate compositions.

16. A preparation according to any of claims 13 to 15 which comprises also a composition containing human chorionic gonadotropin for use in the treatment for inducing ovulation.

17. A preparation according to any of claims 13 to 16 in which the antagonist is in the form of a composition suitable for administering a daily dose of 1.0 to 3.0 mg antagonist per kg body weight.

18. A preparation according to any of claims 13 to 17 in which the antagonist is (Ac-pClPhe$^1$, pClPhe$^2$, D-Trp$^3$, D-Arg$^6$, D-Ala$^{10}$)GnRH, HCl.

**Revendications**

1. Utilisation d'un antagoniste de la gonadolibérine pour préparer une composition pharmaceutique pour l'emploi avec une composition pharmaceutique comprenant une gonatrophonie choisie parmi a) les gonadotrophines humaines de femmes ménopausées et b) la FSH sensiblement dépourvue de LH, dans le traitement de la stérilité de la femme ou des femelles pour éviter la variabilité des oestrogènes, dans lequel traitement la composition d'antagoniste est administrée en une quantité efficace conjointement avec la composition de gonadotrophine.

2. Utilisation d'une gonadotrophine choisie parmi a) les gonadotrophines humaines de femmes ménopausées et b) la FSH sensiblement dépourvue de LH, pour préparer une composition pharmaceutique pour l'utilisation avec une composition pharmaceutique comprenant un antagoniste de la gonadolibérine, dans le traitement de la stérilité de la femme ou des femmes, pour éviter la variabilité des oestrogènes, dans lequel traitement la composition de l'antagoniste est administrée en une quantité efficace conjointement avec la composition de gonadotrophine.

3. Utilisation d'un antagoniste de la gonadolibérine et d'une gonadotrophine choisie parmi a) les gonadotrophines humaines de femmes ménopausées et b) la FSH sensiblement dépourvue de LH, pour préparer une composition pharmaceutique pour l'utilisation dans le traitement de la stérilité de la femme ou des femelles, pour éviter la variabilité des oestrogènes, par administration conjointe d'une quantité efficace d'une composition contenant un antagoniste de la gonadostimuline et d'une composition comprenant une gonadotrophine.

4. Utilisation selon l'une quelconque des revendications précédentes, dans laquelle la composition de gonadotrophine comprend des gonadotrophines humaines de femmes ménopausées constituées de quantités approximativement égales de FSH et de LH.

5. Utilisation selon l'une quelconque des revendications précédentes, dans laquelle l'antagoniste et la gonadotrophine utilisés conjointement sont dans des compositions séparées.

6. Utilisation selon l'une quelconque des revendications précédentes, dans laquelle la composition contenant l'antagoniste est sous une forme appropriée à l'administration d'une posologie journalière de 1,0 à 3,0 mg de l'antagoniste/kg de poids corporel.

7. Utilisation selon l'une quelconque des revendications précédentes, dans laquelle l'antagoniste est (Ac-pClPhe$^1$ ,pClPhe$^2$ , D-Trp$^3$ ,D-Arg$^6$ ,D-Ala$^{10}$ )GnRH.HCl.

8. Utilisation selon l'une quelconque des revendications précédentes, dans laquelle la composition contenant une gonadotrophine est sous une forme appropriée à l'administration d'une dose journalière contenant 75 à 225 UI de FSH.

9. Utilisation selon la revendication 8, dans laquelle la composition est sous une forme appropriée à l'administration d'une posologie journalière de 1,5 à 4,0 UI de FSH/kg de poids corporel.

10. Utilisation selon l'une quelconque des revendications précédentes, dans laquelle l'une ou les deux compositions est/sont sous une forme appropriée à l'administration parentérale.

11. Utilisation selon l'une quelconque des revendications précédentes, dans laquelle le traitement comprend l'utilisation d'une composition pharmaceutique contenant la gonadotrophine chorionique humaine pour l'induction de l'ovulation.

12. Utilisation selon l'une quelconque des revendications précédentes, dans laquelle le traitement est destiné à la fécondation in vitro.

13. Préparation pharmaceutique contenant une gonadotrophine choisie parmi a) les gonadotrophines humaines de femmes ménopausées et b) la FSH sensiblement dépourvue de LH et un antagoniste de la gonadolibérine sous forme d'une préparation combinée pour l'utilisation conjointe pour éviter la variabilité des oestrogènes dans un procédé de traitement de la stérilité des femmes ou des femelles, dans lequel traitement la composition d'antagoniste est administrée en une quantité efficace conjointement à la composition de gonadotrophine.

14. Préparation selon la revendication 13, dans laquelle la composition de gonadotrophine comprend des gonadotrophines humaines de femmes ménopausées constituées de quantités approximativement égales de FSH et de LH.

15. Préparation selon l'une quelconque des revendications 13 ou 14, dans laquelle l'antagoniste et la gonadotrophine sont dans la même composition ou dans des compositions séparées.

16. Préparation selon l'une quelconque des revendications 13 à 15, qui comprend également une composition contenant de la gonadotrophine chorionique humaine pour l'utilisation dans le traitement d'induction de l'ovulation.

17. Préparation selon l'une quelconque des revendications 13 à 16, dans laquelle l'antagoniste est sous forme d'une composition convenant à l'administration d'une posologie journalière de 1,0 à 3,0 mg d'antagoniste/kg de poids corporel.

18. Préparation selon l'une quelconque des revendications 13 à 17, dans laquelle l'antagoniste est (Ac-pClPhe$^1$, pClPhe$^2$, DTrp$^3$, D-Arg$^6$, D-Ala$^{10}$)GnRH.HCl.

**Patentansprüche**

1. Verwendung eines GRH-(Gonadotropin-releasing-Hormon)-Antagonisten zur Herstellung einer pharmazeutischen Zusammensetzung, welche ihrerseits für eine Verwendung gemeinsam mit einer pharmazeutischen Zusammensetzung bestimmt ist, welche letztgenannte Gonadotropine enthält, welche aus
   a) Human-Menopausen-Gonadotropinen und
   b) FSH (follikelstimulierendem Hormon), welches im wesentlichen frei von LH (Luteinisierungshormon) ist,
ausgewählt sind, bei der Behandlung von weiblicher Unfruchtbarkeit, um die Östrogen-Schwankungen zu unterdrücken, bei welcher Behandlung die Antagonisten-Zusammensetzung in einer wirksamen Menge gemeinsam mit der Gonadotropin-Zusammensetzung verabreicht wird.

2. Verwendung von Gonadotropinen, welche aus
   a) Human-Menopausen-Gonadotropinen und
   b) FSH, welches im wesentlichen frei von LH ist,
ausgewählt sind, zur Herstellung einer pharmazeutischen Zusammensetzung, welche für eine Verwendung gemeinsam mit einer pharmazeutischen Zusammensetzung bestimmt ist, welche einen GRH-Antagonisten enthält, bei der Behandlung von weiblicher Unfruchtbarkeit, um die Östrogen-Schwankungen zu unterdrücken, bei welcher Behandlung die Antagonisten-Zusammensetzung in einer wirksamen Menge gemeinsam mit der Gonadotropin-Zusammensetzung verabreicht wird.

3. Verwendung eines GRH-Antagonisten und von Gonadotropinen, welche aus
   a) Human-Menopausen-Gonadotropinen und
   b) FSH welches im wesentlichen frei von LH ist,
ausgewählt sind, zur Herstellung einer pharmazeutischen Zusammensetzung, welche für die Behandlung von weiblicher Unfruchtbarkeit bestimmt ist, um die Östrogen-Schwankungen durch gemeinsame Verabreichung einer wirksamen Menge einer einen GRH-Antagonisten enthaltenden Zusammensetzung und einer Gonadotropine enthaltenden Zusammensetzung zu unterdrücken.

4. Verwendung nach einem der Ansprüche 1 bis 3, wobei die Gonadotropin-Zusammensetzung Human-Menopausen-Gonadotropine enthält, welche ihrerseits annähernd gleiche Mengen von FSH und LH enthalten.

5. Verwendung nach einem der Ansprüche 1 bis 4, wobei der Antagonist und die Gonadotropine für den gemeinsamen Gebrauch in getrennten Zusammensetzungen vorliegen.

6. Verwendung nach einem der Ansprüche 1 bis 5, wobei die den Antagonisten enthaltende Zusammensetzung in einer Form vorliegt, welche für die Verabreichung einer täglichen Dosis von 1,0 bis 3,0 mg des Antagonisten je kg Körpergewicht geeignet ist.

7. Verwendung nach einem der Ansprüche 1 bis 6, wobei der Antagonist (Ac-pClPhe$^1$, pClPhe$^2$, D-Trp$^3$, D-Arg$^6$, D-Ala$^{10}$)GRH.HCl ist.

8. Verwendung nach einem der Ansprüche 1 bis 7, wobei die Gonadotropine enthaltende Zusammensetzung in einer Form vorliegt, welche für die Verabreichung einer täglichen Dosis geeignet ist, welche 75 bis 225 IE an FSH enthält.

9. Verwendung nach Anspruch 8, wobei die Zusammensetzung in einer Form vorliegt, welche für die Verabreichung einer täglichen Dosis geeignet ist, die 1,5 bis 4,0 IE an FSH je kg Körpergewicht enthält.

10. Verwendung nach einem der Ansprüche 1 bis 9, wobei der Zusammensetzung irgendeine der beiden Zusammensetzung oder jeder der beiden Zusammensetzungen in einer Form zur Verfügung gestellt wird, welche für eine parenterale Verabreichung geeignet ist.

11. Verwendung nach einem der Ansprüche 1 bis 10, wobei die Behandlung die Verwendung einer pharmazeutischen Zusammensetzung umfaßt, welche HCG (Human-Choriongonadotropin) enthält, um die Ovulation zu induzieren.

12. Verwendung nach einem der Ansprüche 1 bis 11, wobei die Behandlung für eine in-vitro-Befruchtung bestimmt ist.

13. Pharmazeutische Präparation, welche Gonadotropine, welche aus
   a) Human-Menopausen-Gonadotropinen und
   b) FSH, welches im wesentlichen frei von LH ist,
ausgewählt sind, und einen GRH-Antagonisten enthält, als eine kombinierte Präparation für den gemeinsamen Gebrauch zur Unterdrükkung der Östrogenschwankungen in einem Verfahren zur Behandlung von weiblicher Unfruchtbarkeit, bei welcher Behandlung die Antagonisten-Zusammensetzung in einer wirksamen Menge gemeinsam mit der Gonadotropin-Zusammensetzung verabreicht wird.

14. Präparation nach Anspruch 13, wobei die Gonadotropin-Zusammensetzung Human-Menopausen-Gonadotropine enthält, welche ihrerseits annähernd gleiche Menge von FSH und LH enthält.

15. Präparation nach Anspruch 13 oder 14, wobei der Antagonist und die Gonadotropine in der gleichen oder in getrennten Zusammensetzungen vorliegen.

16. Präparation nach einem der Ansprüche 13 bis 15, welche auch eine Zusammensetzung mit einem Gehalt an HCG umfaßt, welche ebenfalls für den Gebrauch bei der Behandlung bestimmt ist und dazu dient, eine Ovulation zu induzieren.

17. Präparation nach einem der Ansprüche 13 bis 16, wobei der Antagonist in der Form einer Zusammensetzung vorliegt, welche für die Verabreichung einer täglichen Dosis von 1,0 bis 3,0 mg des Antagonisten je kg Körpergewicht geeignet ist.

18. Präparation nach einem der Ansprüche 13 bis 17, wobei der Antagonist (Ac-pClPhe[1], pClPhe[2], D-Trp[3], D-Arg[6], D-Ala[10])GRH.HCl ist.

FIG.1.

FIG. 2.

FIG.3